# EUROPEAN PATENT APPLICATION

(11) **EP 4 321 020 A1**
(43) Date of publication of application: **14.02.2024**
(21) Application number: 22784724.1
(22) Date of filing: 08.04.2022
(51) Int. Cl.: A01K 67/033, A23L 33/10, A61K 35/12, A61K 39/00, A61K 39/12, A61P 37/04, C12N 5/10, C12N 15/34, C12N 15/40, C12N 15/866

(54) **ORAL VACCINE COMPOSITION**

(30) Priority: 09.04.2021 JP 2021066427
(71) Applicant: Kaico Ltd., Fukuoka-shi, Fukuoka 819-0388 (JP); Kagoshima University, Kagoshima-shi, Kagoshima 890-8580 (JP); Kyushu University, National University Corporation, Nishi-ku Fukuoka-shi Fukuoka 819-0395 (JP)
(72) Inventor: YAMATO Kenta, Fukuoka-shi, Fukuoka 819-0388 (JP); MIYATA Takeshi, Kagoshima-shi, Kagoshima 890-8580 (JP); KUSAKABE Takahiro, Fukuoka-shi, Fukuoka 819-0395 (JP); LEE Jae Man, Fukuoka-shi, Fukuoka 819-0395 (JP); MASUDA Akitsu, Fukuoka-shi, Fukuoka 819-0395 (JP)
(74) Representative: J A Kemp LLP
(86) International application number: PCT/JP2022/017328
(87) International publication number: WO 2022/215742

(57) **Abstract**

A method of easily producing an immunogenic pupa for oral administration and a pupa for oral administration produced by the method are provided. The method is a method of producing a pupa for oral administration, including a step of infecting a larva or pupa of an insect capable of being baculovirus-infected with a recombinant baculovirus having DNA encoding an antigen protein introduced therein and freeze-drying a pupa having pupated from the infected larva or the infected pupa.

## Description

### Technical Field

The present invention relates to a pupa for oral administration, an oral vaccine composition, and methods of producing them.

### Background Art

Baculovirus is a nucleopolyhedrovirus (NPV) that infects insects as its main host, and forms a crystalline protein referred to as "polyhedrin" in the nucleus of infected cells during the proliferation process. As a method of producing a protein of interest using a baculovirus-silkworm (*Bombyx mori*) system, there is a method in which a gene encoding a protein of interest is introduced into a baculovirus, and a larva or pupa of a silkworm is inoculated with the resulting recombinant baculovirus, thereby allowing the silkworm to produce the protein of interest (Non Patent Literature 1). In a case where a vaccine is produced using a baculovirus-silkworm system as described above, it is advantageous in that a large amount of a protein of interest can be produced.

However, in the case of producing a vaccine, in general, steps such as extraction and/or purification of an antigen protein produced by a silkworm are required.

Therefore, it is desired to develop a method of producing a vaccine more easily using a baculovirus-silkworm system.

### Citation List

### Non Patent Literature

Non Patent Literature 1 : Maeda et al., "Production of human α-interferon in silkworm using a baculovirus vector." Nature, 315, 592-594 (1985)

### Summary of Invention

### Technical Problem

An object of the present invention is to provide a method of easily producing an immunogenic pupa for oral administration and a pupa for oral administration produced by the method. Another object of the present invention is to provide an oral vaccine composition comprising the pupa for oral administration and a method of producing the same.

### Solution to Problem

The present inventors have surprisingly found that silkworm pupae can remain immunogenic after being infected with a recombinant baculovirus having DNA encoding an antigen protein introduced therein and then freeze-dried. This has led to the completion of the present invention.

Specifically, the present invention involves each of the following inventions.
(1) A pupa of an insect capable of being baculovirus-infected, which is infected with a recombinant baculovirus having DNA encoding an antigen protein introduced therein and then freeze-dried.
(2) A cell capable of being baculovirus-infected, which is infected with a recombinant baculovirus having DNA encoding an antigen protein introduced therein and then freeze-dried.
(3) An oral vaccine composition, comprising the pupa according to (1) and/or the cell according to (2).
(4) The oral vaccine composition according to (3), further comprising a solution containing an adjuvant.
(5) The oral vaccine composition according to (3) or (4), wherein the pupa is contained in an amount of at least 20% by weight based on the total weight of the composition.
(6) A functional food, containing the pupa according to (1) and/or the cell according to (2).
(7) A method of producing a pupa for oral administration, comprising a step of infecting a larva or pupa of an insect capable of being baculovirus-infected with a recombinant baculovirus having DNA encoding an antigen protein introduced therein and freeze-drying a pupa having pupated from the infected larva or the infected pupa.
(8) A method of producing a cell for oral administration, comprising a step of infecting a cell capable of being baculovirus-infected with a recombinant baculovirus having DNA encoding an antigen protein introduced therein and freeze-drying the infected cell.
(9) A method of producing an oral vaccine, comprising a step of infecting a larva or pupa of an insect capable of being baculovirus-infected with a recombinant baculovirus having DNA encoding an antigen protein introduced therein and freeze-drying a pupa having pupated from the infected larva or the infected pupa.
(10) A method of producing an oral vaccine, comprising a step of infecting a cell capable of being baculovirus-infected with a recombinant baculovirus having DNA encoding an antigen protein introduced therein and freeze-drying the infected cell.
(11) The method according to (7) or (9), wherein the insect is *Bombyx mori.*
(12) The method according to (8) or (10), wherein the cell is from *Spilosoma imparilis*, *Antheraea pernyi*, *Bombyx mori*, *Spodoptera frugiperda*, or *Trichoplusia ni.*
(13) A method of inducing immunity to an antigenic protein in a subject by administering the pupa according to (1), the cell according to (2), the oral vaccine composition according to any one of (3) to (5), or the food according to claim 6 to the subject.

### Advantageous Effects of Invention

According to the present invention, a method of easily producing an immunogenic pupa for oral administration and an oral vaccine composition comprising the same is provided. In addition, the thus produced pupa for oral administration can be used as is for administration, and therefore, extraction/purification of an antigen protein or a treatment with a drug, for example, is not required.

### Brief Description of Drawings

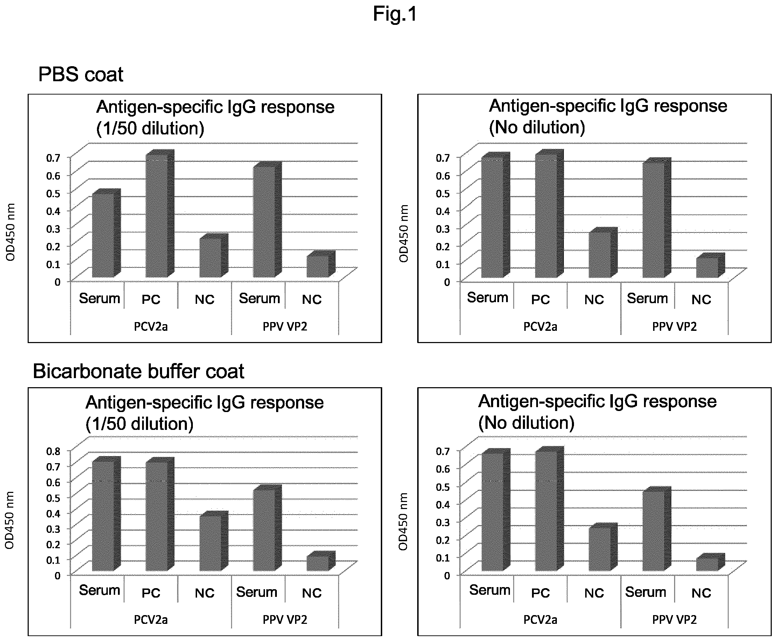

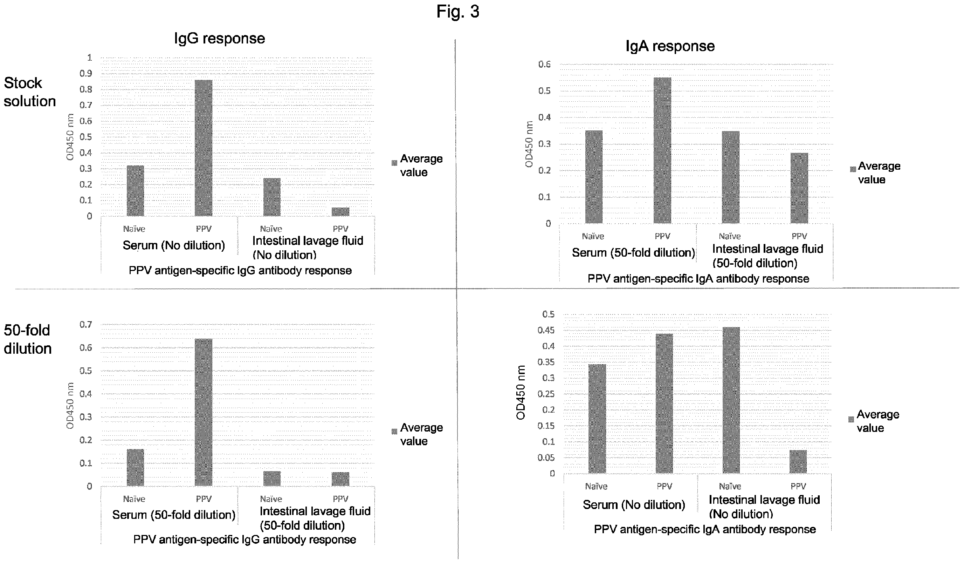

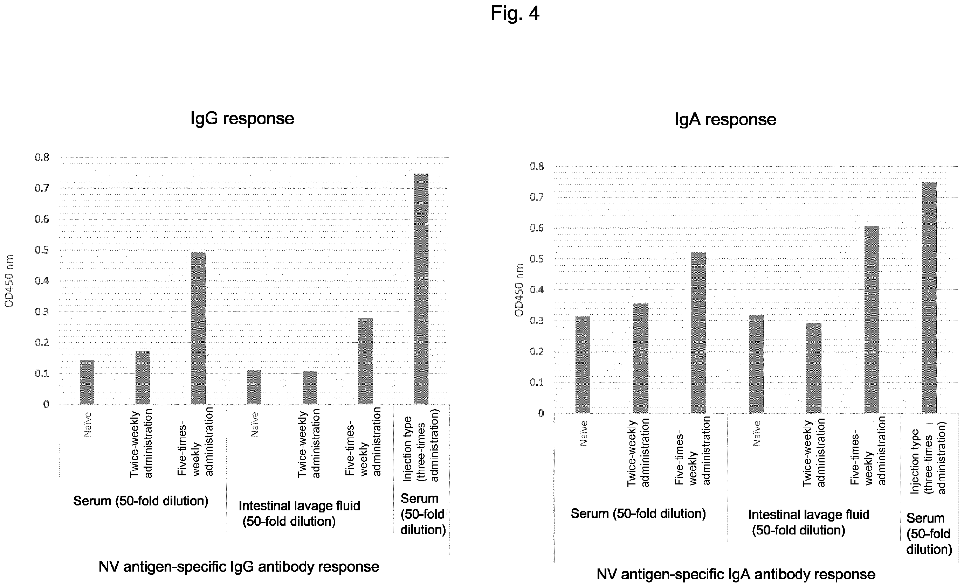

Figure 1 is a view showing the results of confirming the immunogenicity of pupae expressing PCV2a and that of pupae expressing PPV VP2 separately by ELISA using the sera of pupa-fed mice. The upper panels of Figure 1 show the results for PBS coat, and the lower panels of Figure 1 show the results for bicarbonate buffer coat. "No dilution" indicates the results of using the primary antibody as is as a stock solution, and "1/50 dilution" indicates the results of using a 50-fold dilution of the primary antibody.

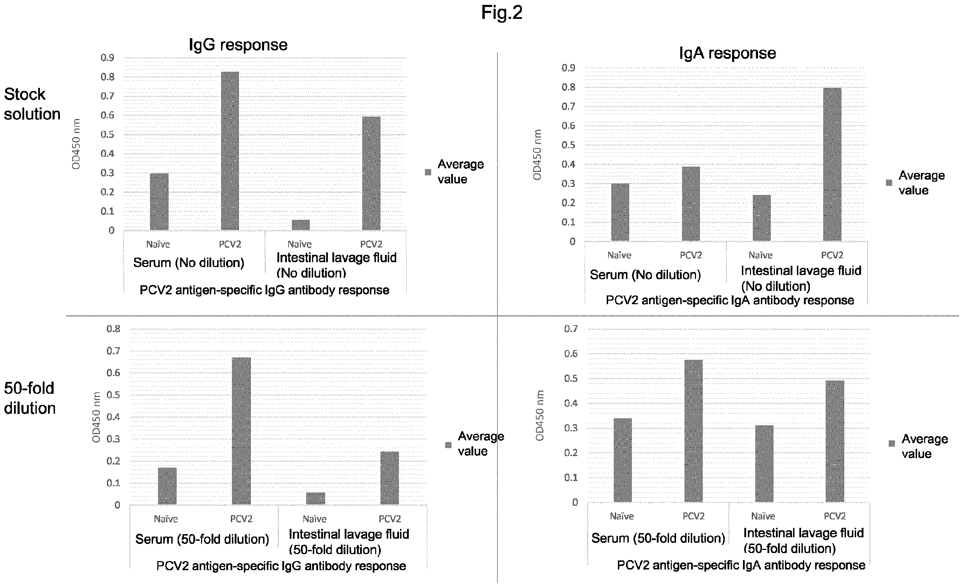

Figure 2 is a view showing antigen-specific IgG antibody or IgA antibody production response to the PCV2 ORF2 antigen.
   Upper left: IgG antibody production response of serum (stock solution) and intestinal lavage solution (stock solution) (OD 450 nm)
   Lower left: IgG antibody production response of serum (50-fold dilution) and intestinal lavage fluid (50-fold dilution) (OD 450 nm)
   Upper right: IgA antibody production response of serum (stock solution) and intestinal lavage solution (stock solution) (OD 450 nm)
   Lower right: IgA antibody production response of serum (50-fold dilution) and intestinal lavage fluid (50-fold dilution) (OD 450 nm)
   Each value shows the average value of N = 3. Serum and intestinal lavage fluid in the non-administration group are referred to as "Naive."
Figure 3 is a view showing antigen-specific IgG antibody or IgA antibody production response to the PPV VP2 antigen.
   Upper left: IgG antibody production response of serum (stock solution) and intestinal lavage solution (stock solution) (OD 450 nm)
   Lower left: IgG antibody production response of serum (50-fold dilution) and intestinal lavage fluid (50-fold dilution) (OD 450 nm)
   Antigen-specific production response to the PPV VP2 antigen
   Upper right: IgA antibody production response of serum (stock solution) and intestinal lavage solution (stock solution) (OD 450 nm)
   Lower right: IgA antibody production response of serum (50-fold dilution) and intestinal lavage fluid (50-fold dilution) (OD 450 nm)
   Each value shows the average value of N = 3. Serum and intestinal lavage fluid in the non-administration group are referred to as "Naive."
Figure 4 is a view showing antigen-specific antibody production response to the NV VP1 antigen.
   Left panel: IgG antibody production response of serum (50-fold dilution) and intestinal lavage fluid (50-fold dilution) (OD 450 nm)
   Right panel: IgA antibody production response of serum (50-fold dilution) and intestinal lavage fluid (50-fold dilution) (OD 450 nm)
   Each value shows the average value of N = 6. Serum and intestinal lavage fluid in the non-administration group are referred to as "Naive."

The group in which pupae were administered twice a week is referred to as the "twice-weekly administration group," and the group in which pupae were administered five times a week is referred to as the "five-times-weekly administration group."

### Description of Embodiments

### 1. Overview

In the case of producing a vaccine antigen against an infectious disease using a heterologous silkworm protein expression system, the vaccine antigen can be produced by preparing a recombinant baculovirus having a vaccine antigen gene from a target pathogenic microorganism (e.g., a virus) inserted therein and inoculating silkworm pupae or larvae such that the virus propagates in the silkworm individuals. For a vaccine administered by ordinary injection, it is necessary to purify some vaccine antigen protein. Then, in order to purify it, it is necessary to combine a plurality of chromatograms such as affinity purification, ion exchange purification, and ammonium sulfate precipitation. However, if it is possible to administer, as the vaccine antigen, a silkworm pupa itself, in which the vaccine antigen is expressed, the cost of purification can be reduced, and the labor involved in administration by injection can be expected to be reduced.

In the present invention, pupae and cells having high antigenicity were successfully acquired by freeze-drying pupae expressing a vaccine antigen or cells expressing a vaccine antigen. Then, these pupae or cells were fed to mice in order to verify whether the antibody production response was actually enhanced. As a result, it was found that the antibody production response was remarkably enhanced against two types of porcine virus antigens and one type of human virus antigen, and that even antibody induction in the blood and intestinal tract was possible.

The present invention is based on the above findings.

The present invention provides a method of producing a pupa for oral administration, comprising a step of infecting a larva or pupa of an insect capable of being baculovirus-infected with a recombinant baculovirus having DNA encoding an antigen protein introduced therein and freeze-drying a pupa having pupated from the infected larva or the infected pupa. In addition, the present invention provides a method of producing a cell for oral administration, comprising a step of infecting a cell capable of being baculovirus-infected with a recombinant baculovirus having DNA encoding an antigen protein introduced therein and freeze-drying the infected cell.

Both of the pupa and the cell after lyophilization can be used as an oral vaccine.

### 2. Recombinant Baculovirus Having DNA Encoding Antigen Protein Introduced Therein

As used herein, an antigenic protein means a protein that exhibits antigenicity, i.e., immunogenicity.

An antigen is not particularly limited, and various antigens known to be usable can be optionally selected and used. For example, examples of antigens include viral antigens, bacterial antigens, fungal antigens, and parasite antigens. Specific examples thereof include circovirus antigens such as porcine circovirus antigen (PCV), parvovirus antigens such as porcine parvoviral antigen (PPV), norovirus antigen, influenza virus antigen, coronavirus antigens such as SARS-CoV2, herpesvirus antigen, iridovirus antigen, rhabdovirus antigen, birnavirus antigen, malaria antigens such as vivax malaria transmission blocking vaccine candidate antigens (Pvs25, Pvs28), Staphylococcus aureus antigen, Aeromonas antigen, *Mycobacterium tuberculosis* antigen, *Actinobacillus pleuropneumoniae* antigen, *Mycoplasma hyopneumoniae* antigen, *Bordetella bronchiseptica* antigen, *Pasteurella multocida* antigen, *Mycoplasma hyorhinis* antigen, *Haemophilus parasuis* antigen, *Escherichia coli* antigen, *Salmonella choleraesuis* antigen, Streptococcus antigen, influenza virus antigen, AIDS virus antigen, porcine epidemic diarrhea virus antigen, infectious gastroenteritis virus antigen, porcine reproductive and respiratory syndrome virus antigen, Japanese encephalitis virus antigen, Aujeszky's disease virus antigen, Trypanosoma antigen, *Pasteurella multocida* toxin, coccidia antigen, and Theileria antigen. More specific examples of an antigen protein include ORF2 (1-233 (SEQ ID NO: 1)) of PCV2a (GenBank Accession #AF055392) that is an isolate of porcine circovirus type 2 (PCV2) and VP2 (T3 d17) of porcine parvovirus (PPV).

The size of the antigen is not particularly limited and may be, for example, 5 to 1000 kDa, 10 to 500 kDa, or 30 to 200 kDa.

Immunogenicity means a property that an antigen induces antibody production and cell-mediated immunity. The presence or absence of immunogenicity can be evaluated by, for example, the presence or absence of antibody production after antibody administration. Antibody expression can be confirmed by methods known to those skilled in the art, such as enzyme-linked immunosorbent assay (ELISA), Western blotting, immunoprecipitation, flow cytometry and immunohistochemical staining.

A method of introducing DNA encoding the antigen protein into baculovirus can be carried out by a method known to those skilled in the art, for example, the method described in Maeda et al., Nature 315, 592-594 (1985), Y Matsuura et al., Virology, (1989) 173, 674-682, or the like. For instance, DNA encoding the antigen protein (including cDNA) is cloned and integrated into a baculovirus transfer vector, thereby obtaining a recombinant baculovirus transfer vector. Next, using this recombinant baculovirus transfer vector, recombinant baculovirus DNA is obtained by a homologous recombination method or a transposon transfer method. This recombinant baculovirus DNA is introduced into cultured insect cells by a known method such as a lipofection method, thereby obtaining a recombinant baculovirus.

The baculovirus transfer vector is obtained by subcloning a DNA fragment containing the polyhedrin gene of baculovirus genomic DNA into a plasmid, and can be prepared by a known method, or a commercially available one can also be used. Examples of commercially available vectors include pAcYM1, pAcG2T, pAcGP67, and VL1392 (Pharmingen Inc.) and pDEST8 (Invitrogen Corporation).

Examples of type of baculovirus used for preparing a recombinant baculovirus in the prevent invention include *Autographa californica* multiple nucleopolyhedrovirus (AcNPV), *Bombyx mori* nucleopolyhedrovirus (BmNPV), Orgyia pseudotsugata multiple nucleopolyhedrovirus (OpNPV), and Lymantria disper multiple nucleopolyhedrovirus (LdNPV). *Bombyx mori* nucleopolyhedrovirus (BmNPV) is preferable.

### 3. Infection of Larva or Pupa of Insect Capable of Being Baculovirus-Infected

In the present invention, a recombinant baculovirus infects a larva or pupa of an insect capable of being baculovirus-infected or a cell capable of being baculovirus-infected, which serves as a host (collectively referred to as "host").

Examples of an insect serving as a host include lepidopteran insects which are not particularly limited as long as they are suitable for protein expression and can be baculovirus-infected, including *Spilosoma imparilis*, *Antheraea pernyi*, *Bombyx mori*, and *Spodoptera frugiperda.* Such insects may be in either pupal or larval form.

In addition, a cell serving as a host is not particularly limited as long as it is suitable for protein expression and is of a cell line capable of being baculovirus-infected. One feature of being baculovirus-infected is that a glycoprotein called "GP64" is expressed on the cell surface. Therefore, as long as it has such properties, the type of cell is not limited, and cultured cells such as insect cells can be mentioned.

Examples of insect cells include the following.
*Spilosoma imparilis-*derived cells: SpIm
*Antheraea pernyi-*derived cells: Anpe
*Bombyx mori*-derived cells: BmN, BmN4, Oyanagi-2, and Bme21
*Spodoptera frugiperda-*derived cells: Sf9 and Sf21

A method of infecting a host insect or cell with a recombinant baculovirus can be carried out by a method known to those skilled in the art. For example, a recombinant baculovirus obtained in the above step is injected into pupae or larvae. When infecting a host cell, a solution containing a recombinant baculovirus may be added to the cell culture medium.

After the host insect or host cell is infected with the recombinant baculovirus and then bred or cultured for 5 to 8 days, the larva becomes a pupa. Accordingly, the antigen protein is expressed in the host insect or host cell.

Here, in the case of producing a protein of interest using a conventional baculovirus-silkworm system, after the protein of interest is expressed in a silkworm pupa or larva infected with a recombinant baculovirus, the protein of interest is purified by collecting the crushed pupa or body fluid from the larva and performing various purifications. Meanwhile, according to the method of the present invention, there is no need to perform such extraction/purification treatment of the protein of interest, and the pupa or cell expressing the antigen protein can be freeze-dried and then used as is.

### 4. Freeze-Drying of Host Infected with Recombinant Baculovirus

The timing of freeze-drying a host pupa or cell infected with a recombinant baculovirus is preferably after a sufficient amount of the antigen protein is expressed in the host (for example, in a silkworm pupa). In a case where a larva is infected with the baculovirus, it is allowed to grow until it becomes a pupa. Therefore, the timing of freeze-drying is after pupation in a case where the infected silkworm is a larva or at any time after infection in a case where the infected silkworm is a pupa. The pupa infected with the recombinant baculovirus may be freeze-dried in the pupal form without being cut or crushed, or the pupa may be freeze-dried after being cut or crushed.

The expression that a pupa is used "as is" and "in the pupal form" means that the pupa is used or included substantially in the pupal form. For example, also in a case where unintentionally, part of a pupa is lost during the production process, it may be determined that the pupa remains in the pupal form as long as the pupal form is maintained at 90% or more, 80% or more, 70% or more, 60% or more, or 50% or more.

Freeze-drying is a method of drying under reduced pressure (for example, maintaining in a vacuum state) in a frozen state. Freeze-drying can be performed by a method known to those skilled in the art, for example, using a commercially available freeze-dryer. The freezing treatment temperature can be appropriately set by those skilled in the art, but it is, for example, -90°C to -5°C, -80°C to -10°C, or -50°C to -10°C. In addition, pressure reduction conditions can also be adjusted by those skilled in the art, but the pressure is, for example, 2 Pa to 20 Pa, 3 Pa to 20 Pa, or 10 Pa to 20 Pa. The freeze-drying time is, for example, 8 to 36 hours.

Freeze-drying removes most of the moisture from a pupa or cell, leaving the pupa or cell dry. The moisture content of a pupa after freeze-drying may be, for example, 1% by weight or less, 0.5% by weight or less, 0.1% by weight or less, 0.01% by weight or less, or 0.001% by weight or less based on the total weight of the pupa or cell. From the viewpoint of facilitating the maintenance of immunogenicity, the moisture content in a pupa is preferably close to 0% by weight.

Here, in the present invention, after the freeze-drying, the pupa or cell can be optionally heat-treated under pressure. The timing of pressurizing and heating is preferably after the pupa or cell has been sufficiently dried. A step of freeze-drying a host infected with a recombinant baculovirus and a step of heat-treating the host under pressure are continuously performed, meaning that after freeze-drying, heat treatment under pressure can be performed without performing another treatment step therebetween. The timing of pressurized heat treatment after freeze-drying is appropriately adjusted depending on the conditions of the dried silkworm pupa and the like, and the pressurized heat treatment can be performed within, for example, 24 hours, 12 hours, 3 hours, or 1 hour after freeze-drying. It is preferable that the freeze-dried pupa is not cut or crushed, but is heat-treated under pressure in the pupal form.

Pressurized heat treatment means a process of pressurizing in a heated state. The pressurized heat treatment can be performed by a method known to those skilled in the art, and can be performed by, for example, using a commercially available Pressurized heat treatment apparatus (for example, an autoclave). The pressurization conditions can be appropriately set by those skilled in the art, but pressurization may be performed by, for example, at 0.1 MPa to 0.6 MPa or 0.2 MPa to 0.5 MPa. In addition, heating conditions can also be adjusted by those skilled in the art, but the temperature is, for example, 100°C to 150°C or 110°C to 140°C. The time for performing pressurized heat treatment is not particularly limited, but it may be, for example, 3 minutes to 90 minutes, 5 minutes to 60 minutes, or 10 minutes to 30 minutes.

The pupa and cell heat-treated under pressure remains immunogenic. Therefore, in the present invention, there is no need to extract the antigen protein, and it can be used as is for oral administration.

Further, in one aspect of the present invention, the freeze-dried pupa is dried and sponge-like, and thus, a liquid can easily permeate inside the pupa by immersing the pupa in the liquid. Therefore, the production method according to the present embodiment may further include a step of immersing a freeze-dried pupa which, if necessary, is heat-treated under pressure in a liquid. The liquid which permeates is preferably a solution containing an adjuvant because the effect as a vaccine can be easily improved. By immersing the freeze-dried pupa in a solution containing an adjuvant, the solution containing the adjuvant can easily permeate inside the pupa. Since cultured cells that have been freeze-dried form a powdery composition, in a case where cells are used, the freeze-dried cells may be immersed in a solution containing an adjuvant.

The pupa or cell produced by the present invention may be used as is, may be cut or crushed for use, or may be used after allowing a liquid to permeate therethrough as described above.

Examples of the form to be used as is include a form in which the pupa is orally administered as is as a vaccine, and a form in which the pupa is fed as is as an edible form.

Examples of the form to be cut or crushed for use include a form in which the cut or crushed pupa is administered as is, a form in which the crushed pupa is mixed with other orally administrable materials and administered, and a form in which the crushed pupa is mixed with a feed and ingested for edible use. For example, in order to adjust the dosage in consideration of the immunogenicity of the pupa, the pupa may be cut into 1/2, 1/3, or 1/4 sizes, for example. The degree of crushing is not particularly limited as long the pupa is immunogenic. For example, the pupa may be in the powder form. Cutting can be carried out with, for example, food scissors and scissors for laboratory animals, and crushing can be carried out with, for example, a mixer, a hand mill, and a crusher. In the case of using a cell, they can be treated in the same manner as when the pupa is crushed.

Examples of oral administration include intra-oral administration and sublingual administration. As used herein, examples of administration also include a case where a subject spontaneously ingests. In a case where the pupa is administered as is, intra-oral administration of the pupa is preferable, or the subject may spontaneously ingest the pupa.

Examples of additives that can be orally administered include food materials, beverage materials, excipients, thickeners, stabilizers, preservatives, pH adjusters, sweeteners, colorants, emulsifiers, flavors, and pharmaceutical additives described below. The pupa or cell can also be mixed with a food material or a beverage material and administered as a functional food or beverage. A liquid containing a material that can be orally administered is not particularly limited as long as the pupa for oral administration has immunogenicity and is suitable for oral administration, and the liquid may be, for example, water or an aqueous solution.

### 5. Oral Vaccine Composition

The pupa or cell obtained as described above contains a foreign antigen protein and has immunogenicity, and is therefore used as an oral vaccine composition.

The oral vaccine composition may be in the form of a pupa itself, or in any dosage form such as a liquid agent (e.g., syrup or jelly), a powder, granules, tablets, a powdered agent, or capsules. Also, in a case where the cell is used as an oral vaccine composition, it can be in any dosage form such as a powder, granules, tablets, a powdered agent, or capsules.

The above dosage forms are formulated together with pharmaceutical additives by a method usually used in the art. Examples of pharmaceutical additives include adjuvants, carriers for oral administration, diluents, excipients, disintegrants, binders, lubricants, fluidizers, coatings, solubilizers, solubilizing agents, thickeners, dispersants, stabilizers, preservatives, wetting-out agents, tonicity regulators, infiltrators, sweeteners, and flavors. From the viewpoint of improving the efficacy as a vaccine, it is preferable that the pharmaceutical additives include an adjuvant.

Examples of adjuvants include Freund's complete adjuvant, incomplete Freund's adjuvant, *Bordetella pertussis* adjuvant, Ribi adjuvant, lipid A, liposome, aluminum hydroxide, and silica.

The present invention may further include a step of mixing the immunogenic pupae or cell with pharmaceutical additives or a liquid containing them. In the oral vaccine composition of the present invention, the expressed antigen protein is contained in the pupa or cell. Therefore, a component of the immunogenic pupa or cell (e.g., powder of the freeze-dried pupa) can be contained in an amount of, for example, 20% by weight or more, 30% by weight or more, 40% by weight or more, 50% by weight or more, 60% by weight or more, 70% by weight or more, 80% by weight or more, or 90% by weight or more based on the total weight of the vaccine composition.

The liquid used as a pharmaceutical additive is not particularly limited as long as the oral vaccine composition has immunogenicity and is pharmaceutically suitable for oral administration. Examples thereof include an aqueous solutions such as water, saline, or phosphate buffer (PBS).

The immunogenic pupa may be used as is, or it may be cut or crushed for use. In the case of using the cell, it can also be crushed.

In the present invention, in a case where the pupa is used in the pupal form, the step of mixing the pupa with the liquid containing pharmaceutical additives may include a step of immersing the freeze-dried pupa in the liquid. For example, by immersing the pupa in a solution containing an adjuvant, the solution containing the adjuvant can easily permeate inside the pupa. The pupa in which the liquid has permeated may be further cut or crushed so as to obtain an oral vaccine composition. In the case of a cultured cell, it can also be used according to the crushed pupa.

In the case of using the cut or crushed pupa, the step of mixing the pupa with the liquid containing pharmaceutical additives may include a step of dispersing the cut or crushed pupa in the liquid.

A subject to which the pupa for oral administration (vaccine) is administered is not particularly limited, and examples thereof include mammals, birds, reptiles, amphibians, and fish. Mammals include humans and non-human mammals such as mice, rats, pigs, monkeys, bovines, horses, sheep, rabbits, dogs, cats, and goats.

The vaccine for oral administration of the present invention exhibits immunogenicity even when orally administered in the pupal form. Alternatively, the pupa may be cut or crushed for administration, and may be used after allowing a liquid to permeate therethrough as described above.

Examples of the form to be administered in the pupal form include a form in which the pupa for oral administration is orally administered as is as a vaccine, and a form in which the pupa for oral administration is fed as is as an edible form.

Examples of the form to be cut or crushed for administration include a form in which the cut or crushed pupa or cell is orally administered as is, a form in which the cut or crushed pupa or cell is mixed with other orally administrable materials and administered, and a form in which the crushed pupa is mixed with a feed and ingested for edible use. For example, in order to adjust the dosage in consideration of the immunogenicity of the pupa, the pupa may be cut into 1/2, 1/3, or 1/4 sizes, for example. The degree of crushing is not particularly limited as long the pupa is immunogenic. For example, the pupa may be in the powder form.

The dosage can be appropriately set in consideration of the subject of administration and the amount of the antigen protein expressed in the pupa or cell. For example, 1/10 to 2 pupae (0.08 g to 1.6 g) may be administered at a time, and 1/2 to 3/2 pupae (0.2 g to 1.2 g) may be administered at a time. The amount of the antigen protein expressed depends on the type thereof. However, in the case of using PCV2, PPV, and the like, the amount thereof is 0.2 mg to 10 mg with respect to about 800 mg by weight of the pupa, i.e., 0.025% by weight to 1.25% by weight per pupa. According to this, one pupa for oral administration contains 10 times or more of the antigen virus protein of a single dose of a normal injectable vaccine.

The number of times of and duration of administration can also be appropriately set in consideration of the subject of administration and the amount of the antigen protein expressed in the pupa or cell. For example, the number of administration may be once to five times a day, once to three times a day, or once a day. For example, the duration of administration may be 1 to 7 days per week, 1 to 5 days per week, or 1 to 4 days per week. The administrations may be repeated for 2 to 10 weeks, for 1 to 7 weeks, for 2 to 5 weeks, or for 2 to 4 weeks, for example.

When administering the vaccine, it is desirable to cause the subject to fast for about 1 to 3 hours before administration (before feeding).

Specifically, for example, the administration can be carried out as follows.
Administration target: For mice
Dosage: 1/4 to 1 pupa (0.2 g to 1.6 g) administered once a day (feeding)
Duration: 4 to 5 doses per week repeated for 3 to 4 weeks

Further, thereafter, booster immunization may be performed by similarly administering the pupae every 1 to 4 weeks, preferably every 1 to 2 weeks.

The pupae and cells of the present invention can also be concomitantly used with or be used in combination with other vaccines. Other vaccines may have immunogenicity to the same antigenic protein as the pupae or cells of the present invention, or may have immunogenicity to different antigenic proteins. In a case where other vaccines may have immunogenicity to the same antigenic protein as the pupae or cells, the pupae of the present invention may be administered, for example, for booster immunization of the other vaccines described above.

### 6. Food

The food of the present invention is immunogenic because it contains pupae or cells.

The food containing pupae for oral administration is not particularly limited, and examples thereof include animal foods and vegetable foods. The food can also be in the form of animal feed.

The form in which the pupa for oral administration is contained in a food is not particularly limited as long as the food is immunogenic, and pupae having immunogenicity may be used as is or in a cut form, or in a crushed form.

A subject which is fed with the food is not particularly limited, and examples thereof include mammals, birds, reptiles, amphibians, and fish as described above. Mammals include humans and non-human mammals such as mice, rats, pigs, monkeys, bovines, horses, sheep, rabbits, dogs, cats, and goats.

### 7. Method of Inducing Immunity

By administering the above-described silkworm pupae for oral administration or an oral vaccine composition or food containing the same to the subject, immunity can be induced in the subject.

The administration and the like are as described above.

The induction of immunity in the subject and the degree of immunity can be confirmed by a method well known to those skilled in the art, such as measurement of antibody production or antibody titer in the subject.

### Examples

Hereinafter, the present invention will be described in more detail with reference to the Examples below, but the technical scope of the present invention is not limited to these Examples.

### [Example 1] Production of Pupae for Oral Administration of Livestock-Virus Vaccine Antigen

DNA encoding ORF2 (1-233) of PCV2a (GenBank Accession No. LC381288) that is an isolate of porcine circovirus type 2 (PCV2) or DNA encoding VP2 (T3 d17) of porcine parvovirus (PPV) was introduced into *Bombyx mori* baculovirus by an ordinary method (Purification and characterization of immunogenic recombinant virus-like particles of porcine circovirus type 2 expressed in silkworm pupae. Journal of General Virology, 2018, volume 99, Issue 7, 917-926). The thus obtained recombinant baculovirus was injected into silkworm pupae so as to infect them.

The silkworm pupae were treated at -40°C for 720 minutes in a freeze dryer on Day 8 after injection.

### [Example 2] Feeding Study

Seven-week-old BALB/c mice (n = 3) were used to conduct a feeding study with the pupae for oral administration produced in Example 1.

The mice were forced to fast without water for 2 hours before feeding. Each mouse was isolated and fed ad libitum with the pupae for oral administration. No water was given during feeding of the pupae.

The mice were fed with the pupae according to the schedule in the table below. There were no dietary restrictions other than the pupae during feeding. During the period, 14 pupae were fed per mouse.

**[Table 1]**

| | Pupa |
|---|---|
| Day 1 | Feeding |
| Day 2 | Feeding |
| Day 3 | Feeding |
| Day 4 | Feeding |
| Day 5 | Feeding |
| Day 6 | Feeding withdrawal |
| Day 7 | Feeding withdrawal |
| Day 8 | Feeding |
| Day 9 | Feeding |
| Day 10 | Feeding |
| Day 11 | Feeding |
| Day 12 | Feeding |
| Day 13 | Feeding withdrawal |
| Day 14 | Feeding withdrawal |
| Day 15 | Feeding |
| Day 16 | Feeding |
| Day 17 | Feeding |
| Day 18 | Feeding |
| Day 19 | Feeding withdrawal |
| Day 20 | Feeding withdrawal |
| Day 21 | Feeding withdrawal |
| Day 22 | Sample collection |

After collecting blood under anesthesia on Day 22, serum and intestinal lavage fluid were obtained.

### [Example 3] Confirmation of Specific Antibody Production Response

The immunogenicity of pupae expressing PCV2a and that of pupae expressing PPV VP2 were confirmed separately by ELISAusing the serum and intestinal lavage fluid obtained in Example 2.

The serum and intestinal lavage fluid of the non-administration group are referred to as "Naive."

Coat antigens for ELISA were prepared as follows. The dilution was carried out with PBS.

PCV2a ORF2 (1-233) (0.44 mg/mL) was diluted to 56.8 µL/4943.2 µL (5 µg/mL), used for coating at 50 µL/well, and left standing overnight at 4°C.

PPV VP2 (0.34 mg/mL) was diluted to 73.5 µL/4926.5 µL (5 µg/mL), used for coating at 50 µL/well, and left standing overnight at 4°C.

In addition, various antibodies, substrates, and the like were used as described below.

Primary antibody: Various immune sera and stock solutions of intestinal lavage fluid (without dilution) or 50-fold dilutions thereof (both 50 µL/well).

Secondary antibody: 1/10000 diluted anti-mouse IgG or IgA-HRP in 0.5% BSA was used at 50 µL/well, and the wells were left standing at 37°C for 1 hour.

Blocking: 1% BSA in PBS was used at 200 µL/well, and the wells were left standing at 37°C for 2 hours.

Washing: A 1/50 dilution with 0.5% BSA or a stock solution (undiluted solution) was used at 50 µL/well, and the wells were left standing at 37°C for 2 hours.

Substrate: 1step Turbo TMB-ELISA (Thermo Scientific, 100 µL/well) was used, and the wells were left standing at room temperature for 10 minutes.

Stop solution: The wells were treated with 2M H₂SO₄ (100 µL/well).

The results of measuring the absorbance at 450 nm (OD 450 value) of each well are shown in Figures 1 to 3. The results for PCV2 showed high levels of IgG antibody production response in serum and intestinal lavage fluid. In addition, when the IgA response was also evaluated, antibody production was observed in the serum, but higher production was observed in the intestinal lavage fluid. Not only the response with the stock solution, but also unexpectedly, a sufficient antibody production reaction was observed even with the 50-fold dilution (Figure 2). On the other hand, in the case of PPV, IgG antibody production response and IgA antibody production response were observed in the serum, but no antibody production response was observed in the intestinal lavage fluid (Figure 3). It is considered that these differences are due to differences in the viral properties.

### [Example 4] Production of Pupae for Oral Administration of Human-Virus Vaccine Antigen

DNA encoding VP1 of genotype GII.4 (GenBank Accession No. BAG70500.1) of norovirus (NV) was introduced into *Bombyx mori* baculovirus by an ordinary method. The thus obtained recombinant baculovirus was injected into silkworm pupae so as to infect them.

The silkworm pupae were treated at -40°C for 720 minutes in a freeze dryer on Day 8 after injection.

### [Example 5] Feeding Study

Seven-week-old BALB/c mice (n = 6) were used to conduct a feeding study with the pupae for oral administration produced in Example 1.

The mice were forced to fast without water for 2 hours before feeding. Each mouse was isolated and fed ad libitum with the pupae for oral administration. No water was given during feeding of the pupae.

The mice were fed with the pupae according to the schedule in Tables 2 and 3 below. There were no dietary restrictions other than the pupae during feeding. During the period, 14 or 16 pupae were fed per mouse.

**[Table 2]**

| Five-times-weekly feeding | |
|---|---|
| | Pupa |
| Day 1 | Feeding |
| Day 2 | Feeding |
| Day 3 | Feeding |
| Day 4 | Feeding |
| Day 5 | Feeding withdrawal |
| Day 6 | Feeding withdrawal |
| Day 7 | Feeding |
| Day 8 | Feeding |
| Day 9 | Feeding |
| Day 10 | Feeding |
| Day 11 | Feeding |
| Day 12 | Feeding withdrawal |
| Day 13 | Feeding withdrawal |
| Day 14 | Feeding |
| Day 15 | Feeding |
| Day 16 | Feeding |
| Day 17 | Feeding |
| Day 18 | Feeding |
| Day 19 | Feeding withdrawal |
| Day 20 | Feeding withdrawal |
| Day 21 | Sample collection |

**[Table 3]**

| Twice-weekly feeding | |
|---|---|
| | Pupa |
| Day 1 | Feeding |
| Day 2 | Feeding withdrawal |
| Day 3 | Feeding withdrawal |
| Day 4 | Feeding |
| Day 5 | Feeding withdrawal |
| Day 6 | Feeding withdrawal |
| Day 7 | Feeding withdrawal |
| Day 8 | Feeding |
| Day 9 | Feeding withdrawal |
| Day 10 | Feeding withdrawal |
| Day 11 | Feeding |
| Day 12 | Feeding withdrawal |
| Day 13 | Feeding withdrawal |
| Day 14 | Feeding withdrawal |
| Day 15 | Feeding |
| Day 16 | Feeding withdrawal |
| Day 17 | Feeding withdrawal |
| Day 18 | Feeding |
| Day 19 | Feeding withdrawal |
| Day 20 | Feeding withdrawal |
| Day 21 | Sample collection |

After collecting blood under anesthesia on Day 22, serum and intestinal lavage fluid were obtained.

### [Example 6] Confirmation of Specific Antibody Production Response

The immunogenicity of pupae expressing NV VP1 was confirmed separately by ELISA using the serum and intestinal lavage fluid obtained in Example 5.

The serum and intestinal lavage fluid of the non-administration group are referred to as "Naive."

Coat antigens for ELISA were prepared as follows. The dilution was carried out with PBS.

NV VP2 (1.36 mg/mL) was diluted to 18.4 µL/4981.6 µL (5 µg/mL), used for coating at 50 µL/well, and left standing overnight at 4°C.

In addition, various antibodies, substrates, and the like were used as described below.

Primary antibody: Various immune sera and 50-fold dilutions of intestinal lavage fluid (both 50 µL/well).

Secondary antibody: 1/10000 diluted anti-mouse IgG or IgA-HRP in 0.5% BSA was used at 50 µL/well, and the wells were left standing at 37°C for 1 hour.

Blocking: 1% BSA in PBS was used at 200 µL/well, and the wells were left standing at 37°C for 2 hours.

Washing: A 1/50 dilution with 0.5% BSA was used at 50 µL/well, and the wells were left standing at 37°C for 2 hours.

Substrate: 1step Turbo TMB-ELISA (Thermo Scientific, 100 µL/well) was used, and the wells were left standing at room temperature for 10 minutes.

Stop solution: The wells were treated with 2M H₂SO₄ (100 µL/well).

The results of measuring the absorbance at 450 nm (OD 450 value) of each well are shown in Figure 4. The results in Figure 4 showed high levels of IgG antibody production response in serum and intestinal lavage fluid. In addition, when the IgA response was also evaluated, antibody production was also observed in the serum and the intestinal lavage fluid. Compared with the injectable vaccine, an antibody production reaction was observed at a level comparable to that of the injectable vaccine without the combined administration of an adjuvant. When the antibody production response was evaluated in terms of the number of times of administration for the twice-weekly administration group and the five-times-weekly administration group," enhanced antibody production response was observed in the five-times-weekly administration group.

## Claims

1. A pupa of an insect capable of being baculovirus-infected, which is infected with a recombinant baculovirus having DNA encoding an antigen protein introduced therein and then freeze-dried.

2. A cell capable of being baculovirus-infected, which is infected with a recombinant baculovirus having DNA encoding an antigen protein introduced therein and then freeze-dried.

3. An oral vaccine composition, comprising the pupa according to claim 1 and/or the cell according to claim 2.

4. The oral vaccine composition according to claim 3, further comprising a solution containing an adjuvant.

5. The oral vaccine composition according to claim 3 or 4, wherein the pupa is contained in an amount of at least 20% by weight based on the total weight of the composition.

6. A functional food, containing the pupa according to claim 1 and/or the cell according to claim 2.

7. A method of producing a pupa for oral administration, comprising a step of infecting a larva or pupa of an insect capable of being baculovirus-infected with a recombinant baculovirus having DNA encoding an antigen protein introduced therein and freeze-drying a pupa having pupated from the infected larva or the infected pupa.

8. A method of producing a cell for oral administration, comprising a step of infecting a cell capable of being baculovirus-infected with a recombinant baculovirus having DNA encoding an antigen protein introduced therein and freeze-drying the infected cell.

9. A method of producing an oral vaccine, comprising a step of infecting a larva or pupa of an insect capable of being baculovirus-infected with a recombinant baculovirus having DNA encoding an antigen protein introduced therein and freeze-drying a pupa having pupated from the infected larva or the infected pupa.

10. A method of producing an oral vaccine, comprising a step of infecting a cell capable of being baculovirus-infected with a recombinant baculovirus having DNA encoding an antigen protein introduced therein and freeze-drying the infected cell.

11. The method according to claim 7 or 9, wherein the insect is *Bombyx mori.*

12. The method according to claim 8 or 10, wherein the cell is from *Spilosoma imparilis*, *Antheraea pernyi*, *Bombyx mori*, *Spodoptera frugiperda*, or *Trichoplusia ni.*

13. A method of inducing immunity to an antigenic protein in a subject by administering the pupa according to claim 1, the cell according to claim 2, the oral vaccine composition according to any one of claims 3 to 5, or the food according to claim 6 to the subj ect.
